# EUROPEAN PATENT APPLICATION

(11) **EP 3 766 422 A1**
(43) Date of publication of application: **20.01.2021**
(21) Application number: 18909688.6
(22) Date of filing: 25.05.2018
(51) Int. Cl.: A61B 5/157

(54) **INSPECTION CHIP AND INSPECTION DEVICE**

(30) Priority: 16.03.2018 US 201862643761 P
(71) Applicant: The University of Tokyo, Bunkyo-ku, Tokyo 113-8654 (JP)
(72) Inventor: KIM Beomjoon, Tokyo 113-8654 (JP); TAKAMA Nobuyuki, Tokyo 113-8654 (JP)
(74) Representative: Ernest Gutmann - Yves Plasseraud S.A.S.
(86) International application number: PCT/JP2018/020224
(87) International publication number: WO 2019/176126

(57) **Abstract**

This inspection chip is provided with: a base plate having an inflow hole, a micro flow passage connected to the inflow hole, and a reaction chamber connected to the micro flow passage; a porous micro needle provided at a position overlapping the inflow hole and composed of a biodegradable material; a sensor disposed in the reaction chamber; and a capillary tube pump part which has a fine diameter flow passage, and is provided on the base plate and connected to the reaction chamber.

## Description

### [Technical Field]

The present invention relates to an inspection chip, and more particularly to an inspection chip including a micro needle and an inspection device including the inspection chip.

Priority is claimed on United States provisional patent application 62/643,761 filed in the United States on Mar. 16, 2018, the content of which is incorporated herein by reference.

### [Background Art]

Diabetic patients need to measure their blood glucose several times a day to control their blood glucose level. Self-blood glucose measuring devices currently on the market measure blood glucose by injuring a capillary blood vessel such as a finger with a needle and bringing blood exuding from the wound into contact with a sensor. Since these self-blood glucose measuring devices are accompanied by pain during measurement, it is a heavy burden for diabetic patients who frequently perform measurement.

A micro needle for collecting blood is known as a pain-free, minimally invasive blood-collecting means. Generally, a micro needle for collecting blood is a hollow needle having a length of about 1 mm, an outer diameter of 100 to 300 µm, and an inner diameter of 60 to 100 µm, and a metal such as nickel or a photoresist has been proposed as a material. Patent Document 1 describes a blood monitoring system including a micro needle for collecting blood.

### [Citation List]

### [Patent Literature]

[Patent Document 1]
Japanese Unexamined Patent Application, First Publication No. 2002-78698

### [Summary of Invention]

### [Technical Problem]

The micro needle for blood collection is difficult to manufacture due to its structure and dimensions. Furthermore, if not strong enough, it may break in the body and remain in the skin.

Further, it is important to continuously monitor blood glucose in order to more accurately grasp the condition of a diabetic patient, but the blood monitoring system described in Patent Document 1 does not have a structure for continuously sucking blood, and thus cannot meet this demand. When attempting to perform continuous blood glucose monitoring using the blood monitoring system described in Patent Document 1, various mechanisms such as a pump and a power source for driving the pump are required, which makes the device large and increases the manufacturing cost.

Due to the above circumstances, there is currently no minimally invasive device that allows the patient to easily perform continuous blood glucose monitoring.

An object of the present invention is to provide an inspection chip capable of continuously acquiring and testing blood with minimal invasiveness.

Another object of the present invention is to provide an inspection device which is capable of continuously monitoring substances in blood with minimal invasiveness.

### [Solution to Problem]

A first aspect of the present invention is an inspection chip including: a base plate having an inflow hole, a micro flow passage connected to the inflow hole, and a reaction chamber connected to the micro flow passage; a porous micro needle provided at a position overlapping with the inflow hole and composed of a biodegradable material; a sensor disposed in the reaction chamber; and a capillary tube pump part which has a fine diameter flow passage, and is provided on the base plate and connected to the reaction chamber.

A second aspect of the present invention is an inspection device equipped with the inspection chip of the present invention.

### [Advantageous Effects of Invention]

According to the present invention, blood can be continuously acquired with minimally invasiveness, and various tests and monitoring are possible.

### [Brief Description of Drawings]

FIG 1 is a perspective view of an inspection chip according to an embodiment of the present invention.
FIG. 2 is a plan view schematically showing a base plate of the inspection chip.
FIG. 3 is a cross-sectional view taken along the line I-I of FIG. 2.
FIG. 4 is a cross-sectional view schematically showing a micro needle of the inspection chip.
FIG. 5 is a diagram showing a step in the manufacturing method of the micro needle.
FIG. 6 is a diagram showing a step in the manufacturing method for the micro needle.
FIG. 7 is a diagram showing a step in the manufacturing method for the micro needle.
FIG. 8 is a diagram showing a step in the manufacturing method for the micro needle.
FIG. 9 is a diagram showing a step in the manufacturing method for the micro needle.
FIG. 10 is a diagram showing an example of an inspection device to which the inspection chip is applied.
FIG. 11 is a view showing the back side of the inspection device.
FIG. 12 is a block diagram of the inspection device.

### [Description of Embodiments]

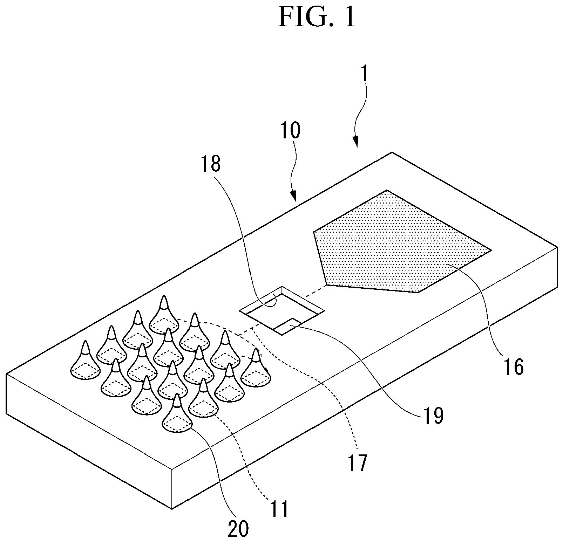

An embodiment of the present invention will be described with reference to FIGS. 1 to 12.

FIG. 1 is a perspective view showing an inspection chip 1 of this embodiment. The inspection chip 1 includes a base plate 10 having a micro flow passage, and a plurality of micro needles 20 and sensors 19 formed on the base plate 10.

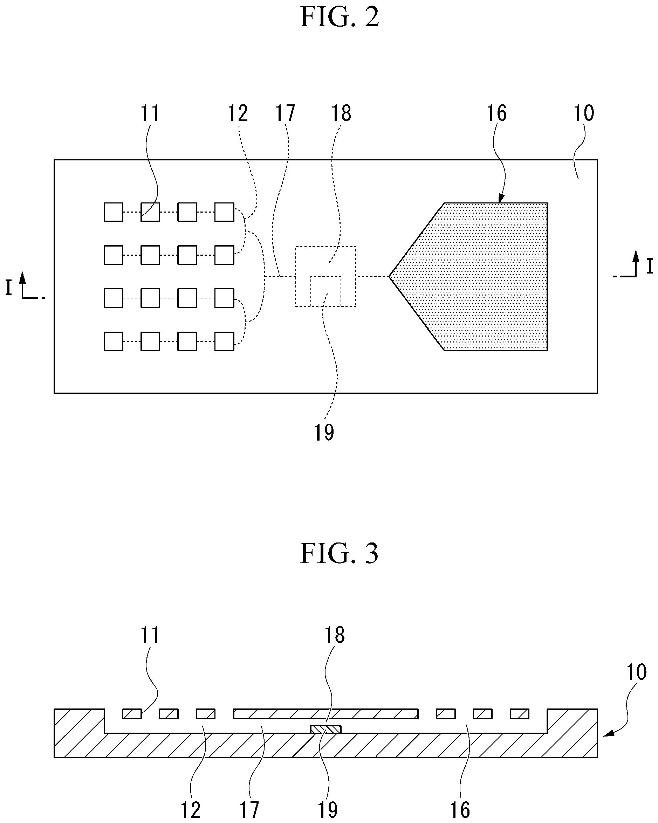

FIG. 2 is a schematic plan view of the base plate 10 before forming the micro needles 20. A plurality of inflow holes 11 are opened in a region on one end side of the base plate 10. A capillary tube pump part 16 is formed in a region on the other end side of the base plate 10. One intermediate channel 17 is formed between the inflow hole 11 and the capillary tube pump part 16.

FIG. 3 is a sectional view taken along the line I-I of FIG. 2. A plurality of micro flow channels 12 are formed in the middle portion of the base plate 10 in the thickness direction. The micro flow passage 12 communicates with each inflow hole 11. The micro flow passages 12 gradually merge as they approach the capillary tube pump part 16, and finally become a single flow passage and are connected to the intermediate flow passage 17.

The capillary tube pump part 16 is composed of a large number of fine diameter flow passages that gradually branch from the intermediate flow passage 17. As a shape that gradually branches, for example, a shape such as a tournament table is an exemplary example. The width and depth of the fine diameter channel may be appropriately set within a range in which a capillary phenomenon is generated, and may be, for example, about 2 to 5 µm.

The upper portion of the capillary tube pump part 16 may be open or may be covered with a cover or the like, but at least the end portion is open to the atmosphere so that the fluid can flow in.

The micro flow passage 12 and the capillary tube pump part 16 of the base plate 10 can be formed by combining photolithography, reactive ion etching, dry etching using xenon difluoride (XeF₂), and the like. From the viewpoint of applying these techniques, a silicon wafer is suitable as the material of the base plate 10.

The width of the intermediate flow passage 17 is widened in the intermediate portion to form a reaction chamber 18. A sensor 19 is installed in the reaction chamber 18. The sensor 19 is at a position where it can come into contact with the fluid flowing through the intermediate flow passage 17.

The specific content of the sensor 19 is appropriately determined according to the item to be measured. For example, in the case of measuring blood glucose level, the electrode part of an electrochemical or optical glucose sensor using glucose oxidase or glucose dehydrogenase can be used.

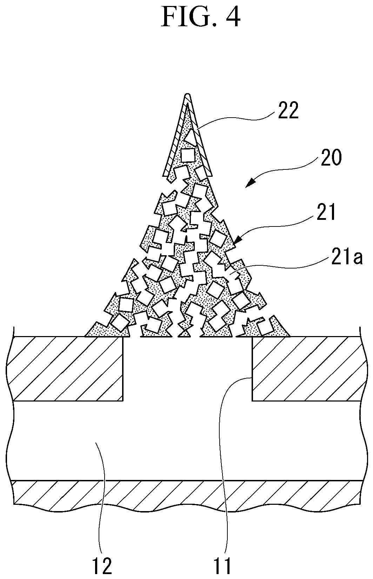

FIG. 4 is a sectional view of the micro needle 20. The micro needle 20 includes a porous main body 21 and a coating 22 that covers the distal end of the main body 21.

The main body 21 is made of a biodegradable material and has a large number of holes 21a on the surface and inside. Examples of biodegradable materials include polylactic acid (PLA), polyglycolic acid (PGA), poly (lactide-co-glycolide) copolymer (PLGA), and the like.

The micro needle 20 has a substantially conical shape or a substantially pyramidal shape, and the diameter or the maximum dimension of the base is, for example, about 50 µm to 200 µm. The height of the micro needle 20 defines the depth of penetration into the skin. In the present embodiment, it is set to 300 µm or more and 1 mm or less in consideration of reaching the dermis and not stimulating pain sensation.

The plurality of holes 21a formed in the main body 21 are partly in communication with each other inside the main body 21. As a result, a communication passage communicating from the side surface to the bottom surface of the main body 21 is formed in the main body 21.

The shape of the hole 21a is not particularly limited. The size of the holes 21a can be appropriately set in consideration of the configuration of the fluid to be collected. For example, in a case in which the fluid contains a solid substance and the solid substance interferes with the measurement performed by the sensor 19, it is possible to make the size of the holes 21a smaller than the solid substance so that the solid substance does not enter the base plate 10.

In a case in which the inspection chip 1 is for measuring blood glucose, the size of the holes 21a can be set to about 30 µm to 60 µm in consideration of the size of the blood cell component, for example.

The coating 22 covers the distal end portion of the main body 21 and constitutes a sharp distal end of the micro needle 20. Examples of the material of the coating 22 include a material having a high affinity for living bodies and having a certain hardness in a dry state, for example, hyaluronic acid.

The manufacturing procedure of the micro needle 20 will be described.

First, the water-soluble particles and the material of the main body 21 are mixed without dissolving the water-soluble particles to prepare a viscous material. The size of the water-soluble particles is the same as the size of the holes 21a formed in the main body 21. The amount of water-soluble particles is determined based on the porosity set in the main body 21. The water-soluble particles are not particularly limited, but sodium chloride is preferable because the particle size can be controlled relatively easily.

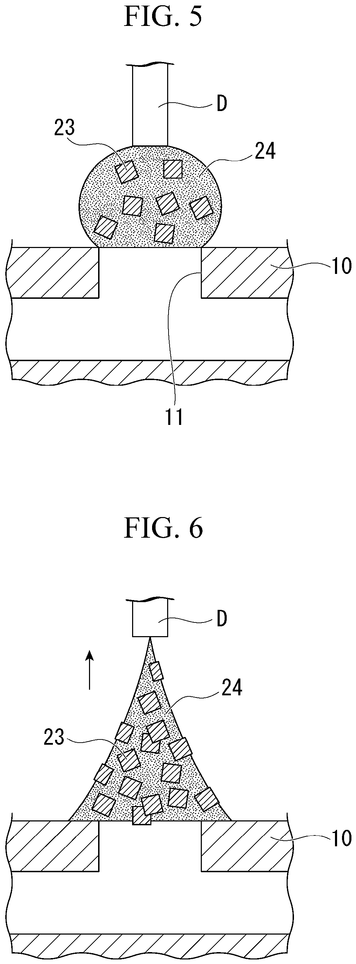

Next, the adjusted viscous material is filled in a dispenser or the like, and the distal end of the dispenser D is brought close to the base plate 10 to gently eject the viscous material, as shown in FIG. 5. As a result, droplets of the viscous material 24 containing the water-soluble particles 23 are arranged on the base plate 10. At this time, the droplets are arranged so as to overlap the inflow holes 11 on the base plate 10.

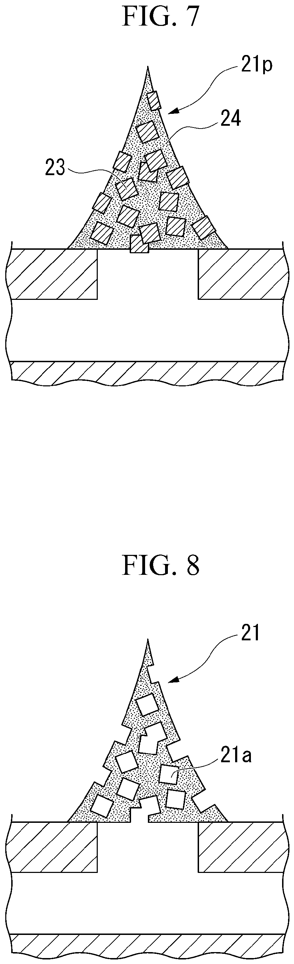

Subsequently, when the dispenser D is slowly pulled up and moved away from the base plate 10, a part of the droplets follow the dispenser D and are pulled up. As a result, the droplet is transformed into a needle-like shape with a sharp upper portion. After the dispenser D is further pulled up and separated from the droplets, the viscous material 24 is dried and solidified to form a master 21p of the main body 21 containing the water-soluble particles 23, as shown in FIG. 7.

Next, the prototype 21p is immersed in water to dissolve the water-soluble particles 23. When the water-soluble particles 23 are removed, as shown in FIG. 8, the parts where the water-soluble particles 23 were present become the holes 21a, and the main body 21 is completed. At this point, in some of the main body 21, the water-soluble particles 23 located at the distal end portion of the prototype 21p are dissolved and removed, so that the distal end portion is missing. Such a main body 21 cannot directly penetrate the skin and does not function as a needle.

Finally, when the distal end of the main body 21 is dipped in a solution of the coating material and pulled up, the coating material is attached so as to cover the distal end of the main body 21, and the distal end has an outer shape like a needle. Even in a case in which the distal end of the main body 21 is missing, the missing material is filled with the coating material, and the distal end shape is almost the same as when the distal end is not missing.

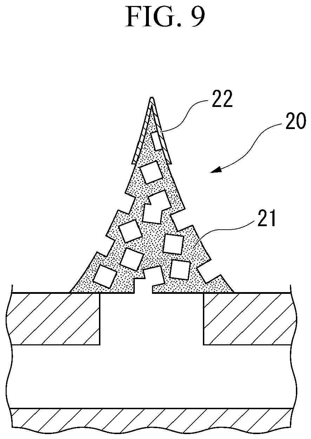

When the attached coating material is dried, as shown in FIG. 9, the coating 22 covering the distal end of the main body 21 is formed, and the micro needle 20 is completed.

The operation when the inspection chip 1 is used will be described.

When the distal end of the micro needle 20 is pressed against the skin of the user, the micro needle pierces the skin from the distal end and the whole penetrates into the skin. Since the solidified coating 22 is present at the distal end of the micro needle 20, the micro needle 20 has sufficient hardness to penetrate the skin. Due to the length of the main body 21, the main body of the micro needle 20 reaches the dermis and does not stimulate pain sensations. As a result, a state in which blood can be collected from the micro needle 20 is established without causing the user to feel pain.

Since the coating 22 quickly dissolves in the skin, the pores 21a of the main body 21 are exposed in the skin and blood can enter.

The blood that has entered from the holes 21a flows through the communication holes in the main body 21 due to the capillary phenomenon, and enters the inflow hole 11 from the bottom opening of the main body 21. The blood further flows through the micro flow passage 12 to the intermediate channel 17, enters the reaction chamber 18, and comes into contact with the sensor 19. Therefore, the sensor 19 can perform a reaction for measurement on the blood that has entered, and obtain an electric signal obtained as a result.

The blood that has reached the reaction chamber 18 further flows into the capillary tube pump part 16 from the intermediate flow passage 17, and gradually fills the fine diameter flow passage of the capillary tube pump part 16. Since the inflow of blood continues until the capillary tube pump part 16 is completely filled, the sensor 19 can continuously perform measurement until the capillary tube pump part 16 is filled with blood.

As described above, according to the inspection chip 1 of the present embodiment, it is possible to easily perform a continuous blood test by the patient himself, which has been difficult previously, without causing the patient to feel any pain.

Further, since the micro needles 20 are formed of a biodegradable material, even in a case in which the micro needles 20 are broken in the skin due to a user's operation or the like, they are decomposed and absorbed as they are, and no adverse event such as inflammation occurs. Therefore, the load on the living body is small and it is extremely safe.

In the inspection chip 1, blood is continuously collected by the capillary phenomenon that occurs in the capillary tube pump part 16. Therefore, blood can be continuously collected without a mechanical pump or its driving source. As a result, the inspection chip 1 can be made compact and easy to handle, and can be manufactured at low cost.

Further, the time that can be continuously measured by the sensor 19 can be freely adjusted by changing the volume of the capillary tube pump part 16, that is, the area of the capillary tube pump part 16 in a plan view of the base plate 10. Therefore, it is possible to deal with various modes of continuous measurement depending on the target inspection item.

Further, according to the method for manufacturing the micro needle of the present embodiment, after the prototype 21p of the main body 21 is formed of the biodegradable viscous material 24 containing the water-soluble particles 23, the holes 21a are formed by dissolving and removing the water-soluble particles 23. Therefore, by appropriately setting the size of the water-soluble particles to be used, it is possible to control the size of the holes and the porosity in the main body 21 to be formed with extremely high accuracy.

In the study conducted by the inventor using porcine blood, it is known that if there are about 15 micro needles 20 having a pore size of 30 to 60 µm and a porosity of 60 to 80%, it is possible to obtain a sufficient amount of blood for continuous blood glucose measurement. According to the manufacturing method of the present embodiment, it is possible to manufacture micro needles that satisfy such conditions reliably and easily.

Further, since the micro needle 20 has the coating 22 on the distal end, it is not necessary to consider the size of the hole in order to ensure that the distal end of the main body is sharp. Therefore, it is possible to ensure the function as a needle by sharpening the distal end end portion by the coating 22 while setting the optimum pore size and porosity without restriction according to the use conditions. That is, it is possible to achieve both a suitable pore condition and a good skin piercing property at a high level.

The inspection chip 1 of this embodiment can be used more suitably by incorporating it into a predetermined inspection device.

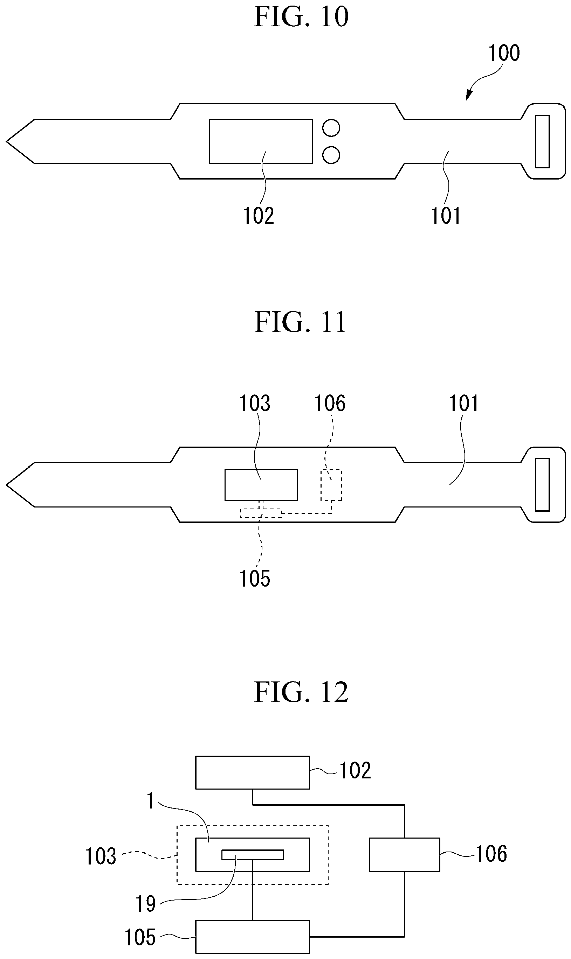

FIG. 10 is a diagram showing an example of an inspection device 100 to which the inspection chip 1 is applied. The inspection device 100 includes a wristband 101 and a display screen 102 provided on the wristband 101.

FIG. 11 is a view showing the back side of the inspection device 100. On the back side of the wristband 101, a cavity 103 for inserting the inspection chip 1 is formed. When the user fits the inspection chip 1 into the cavity 103 and then attaches the wristband 101 to the wrist, the micro needles 20 are pressed against the skin with a certain pressure and pierce the skin. After piercing the skin and starting the blood collection, the wristband 101 holds the micro needle 20 and prevents it from coming off the skin, so that the blood can be stably obtained.

FIG. 12 is a block diagram of the inspection device 100. The inspection device 100 includes a communication unit 105 capable of wireless communication, and a power supply 106 that supplies power to the display screen 102 and the communication unit 105. In a case in which the inspection chip 1 is configured to be applicable to the inspection device 1, a terminal connected to the sensor 19 is formed on the periphery of the inspection chip 1. In this case, the sensor 19 and the communication unit 105 are electrically connected by fitting the inspection chip 1 into the cavity 103, and the electric signal acquired by the sensor 19 can be transmitted to an external terminal such as a computer or a mobile phone.

As another aspect, a configuration may be adopted in which a removable storage medium is provided instead of the communication unit 105, and the electric signal acquired by the sensor 19 is stored in the storage medium. A configuration may be adopted in which both the storage medium and the communication unit are provided, and the electric signal is stored in the storage medium when there is no communicable external terminal nearby. In this case, the storage medium does not have to be removable.

After the measurement is completed, the user removes the inspection chip 1 from the inspection device 100 and discards it. By fitting a new inspection chip 1 into the cavity 103, it is possible to perform repeated inspections easily.

In the above, the wristwatch-type inspection device to be worn on the wrist has been illustrated, but the form of the inspection device is not limited to this, and the shape and attachment site are not particularly limited as long as the micro needle 20 can be held with a constant pressure against the skin. For example, a clip-shaped configuration that is used by sandwiching it between the earlobe and a patch-shaped configuration that includes an adhesive portion and is used by being attached to the skin of the abdomen or chest are used.

Although an embodiment of the present invention and an application example thereof have been described above, the technical scope of the present invention is not limited to the above-described embodiment. It is possible to change the combination of constituent elements, make various changes to each constituent element, or delete the constituent elements beyond the embodiments without departing from the scope of the present invention.

For example, the micro needles in the present invention may be formed by a method other than the method described above. For example, even in a case in which the mold to which the shape of the main body is transferred is filled with a biodegradable material mixed with water-soluble particles and the mold is removed after the base plate 10 is bonded at room temperature without pressure, the micro needle cane be formed on the inflow holes.

In the micro needle of the present invention, the coating mode can be variously changed. In a case in which the coating is made of a material that dissolves quickly in the skin, the coating may cover the entire side of the main body. In a case in which the coating covers only the distal end of the main body, it may not necessarily dissolve quickly in the skin as long as the coating is made of a biodegradable material. Further, the coating may not be provided as long as the distal end of the formed main body has a sharp state due to the relationship between the size of the holes and the size of the main body. That is, the coating is not essential in the micro needle according to the present invention.

Furthermore, a plurality of sets of intermediate flow passages and reaction chambers may be provided, and different sensors may be arranged in each set. In this case, it is possible to continuously perform the inspection of a plurality of items with one inspection chip.

The acquisition target of the inspection chip of the present invention is not limited to blood, and various body fluids that can be acquired subcutaneously can be acquired. For example, interstitial fluid and lymph fluid can be obtained, so that an extremely wide range of tests can be handled by selecting an appropriate sensor and placing it in the reaction chamber.

### [Industrial Applicability]

The present invention can be applied to an inspection chip and an inspection device.

### [Reference Signs List]

- 1: inspection chip
- 10: base plate
- 11: inflow hole
- 12: micro flow passage
- 16: capillary tube pump part
- 18: reaction chamber
- 19: sensor
- 20: micro needle
- 21: main body
- 21a: hole
- 22: coating
- 100: inspection device

## Claims

1. An inspection chip comprising:
a base plate having an inflow hole, a micro flow passage connected to the inflow hole, and a reaction chamber connected to the micro flow passage;
a porous micro needle provided at a position overlapping with the inflow hole and composed of a biodegradable material;
a sensor disposed in the reaction chamber; and
a capillary tube pump part which has a fine diameter flow passage, and is provided on the base plate and connected to the reaction chamber.

2. The inspection chip according to claim 1, wherein the micro needle includes
a main body formed of the biodegradable material and having a plurality of pores, and
a coating that covers at least a distal end of the main body to form the distal end that can be pierced into the skin.

3. The inspection chip according to claim 2, wherein the coating is formed of a material that dissolves in the skin.

4. The inspection chip according to claim 2, wherein, in the main body, the pore size is 30 µm to 60 µm, and the porosity is 60% to 80%.

5. The inspection chip according to claim 1, wherein the biodegradable material comprises at least one selected from the group consisting of polylactic acid, polyglycolic acid, and poly (lactide-co-glycolide) copolymer.

6. An inspection device comprising the inspection chip according to any one of claims 1 to 5.
